**Europäisches Patentamt**

(19) **European Patent Office**

**Office européen des brevets**

(11) Publication number: **0 441 829 B1**

(12) # EUROPEAN PATENT SPECIFICATION

(45) Date of publication of patent specification :
23.09.92 Bulletin 92/39

(51) Int. Cl.⁵ : **A61M 3/02**

(21) Application number : **89912150.3**

(22) Date of filing : **07.11.89**

(86) International application number :
**PCT/SE89/00631**

(87) International publication number :
**WO 90/04983 17.05.90 Gazette 90/11**

(54) **APPARATUS FOR GASTRO-INTESTINAL RINSING AND CLEANING OF HUMAN BEINGS AND ANIMALS.**

(30) Priority : **07.11.88 SE 8804015**
**07.11.88 SE 8804016**

(43) Date of publication of application :
**21.08.91 Bulletin 91/34**

(45) Publication of the grant of the patent :
**23.09.92 Bulletin 92/39**

(84) Designated Contracting States :
**BE DE FR GB LU NL SE**

(56) References cited :
**WO-A-87/01596**
**US-A- 1 949 574**

(56) References cited :
**US-A- 1 949 575**
**US-A- 2 148 541**
**US-A- 2 157 756**
**US-A- 2 313 805**

(73) Proprietor : **MINTHON, Lennart**
**Mariagatan 4**
**S-223 53 Lund (SE)**

(72) Inventor : **MINTHON, Lennart**
**Mariagatan 4**
**S-223 53 Lund (SE)**

(74) Representative : **Ström, Tore et al**
**Ström & Gulliksson AB Studentgatan 1 P.O.**
**Box 4188**
**S-203 13 Malmö (SE)**

## Description

The invention relates to an apparatus for gastro-intestinal irrigation and cleansing of humans and animals comprising a water container having cold and hot water connections for the supply of temperated water to the container, and a nozzle which can be connected by hoses over valve controlled conduits to the water container for supply of water to the nozzle from the water container by gravity and to a drain for discharging flowing material from the nozzle to the drain, the cold water connection being connected via a shut-off valve to the conduit from the water container to the nozzle and via a shut-off valve to the drain, the nozzle hoses being transparent and detachably connected to the valve controlled conduits from the water container and to the drain, respectively, whereby in a conduit connecting the cold water connection to the drain there is provided separate adjustable choke means.

The apparatus is primarily intended for the enema process but can be used also for irrigating and cleansing the stomach.

Apparatus of the kind referred to above for the enema process is disclosed in US-A-I 818 978 and US-A-2 313 805.

The advantage provided by such apparatus is that the treatment takes place in a closed system, and as a consequence thereof the treatment will be considerably less trying physically as well as mentally for the person treated than the conventional enema and, moreover, that the treatment can be carried out faster and considerably more hygienically than the conventional enema with the patient lying the whole time on a couch.

The purpose of the present invention is to improve further the apparatus so that the function thereof can be adapted to the condition of the person to be treated and the very strict hygienic requirements applied nowadays can be satisfied, implying that medical apparatus should be kept free of bacteria.

For the purpose mentioned the apparatus of the kind referred to above according to the invention has obtained the characterizing features of claim I, whereby the drainage through the nozzle can be positively carried out by suction the effect thereof being adjusted to the condition of the patient, and the apparatus can be cleaned and sterilized in a very simple and effective manner.

For detailed description of the invention reference is made to the accompanying drawings in which

FIG I is a functional diagram of an enema apparatus of the invention,
FIG 2 is a diagrammatic perspective view of the apparatus,

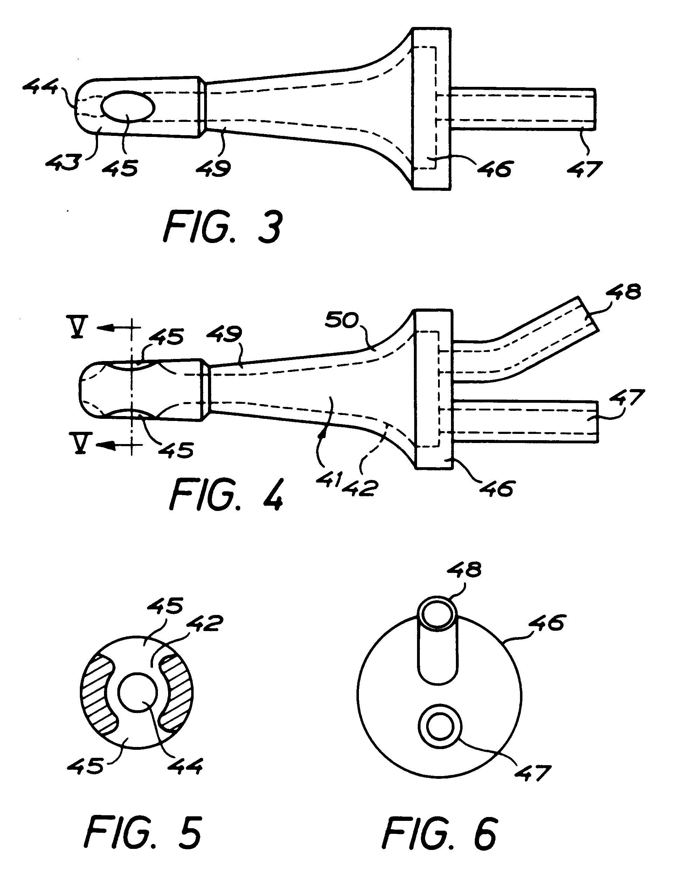

FIG 3 is a side view of the nozzle connected to the apparatus,
FIG 4 is a plan view of the nozzle,
FIG 5 is a cross-sectional view taken along line V-V in FIG 4, and
FIG 6 is an end view of the rear end of the nozzle.

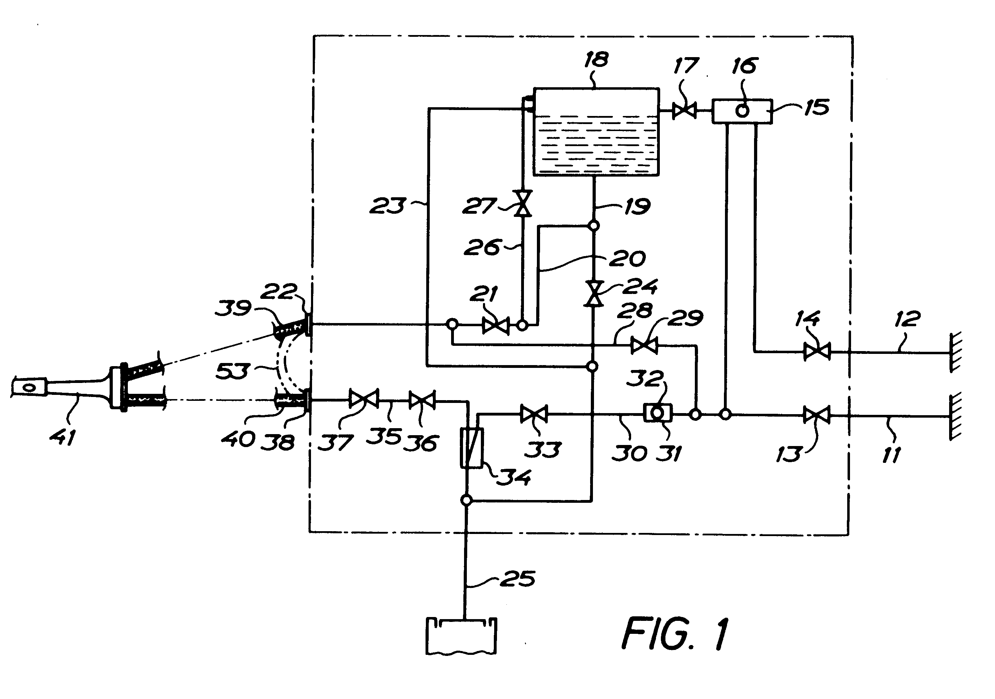

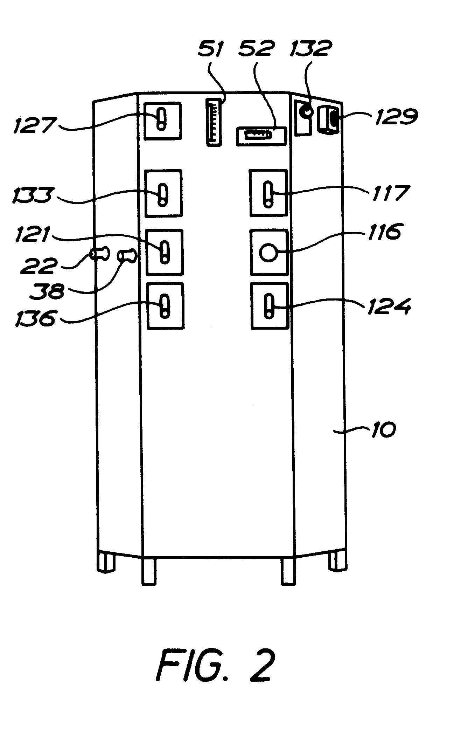

The functional elements shown inside the rectangle indicated by dot-and-dash lines in FIG I, are located inside a housing I0, FIG 2, which preferably is made of stainless sheet and is shaped such that it can be located in a corner of a room so as to require the smallest possible space. The apparatus is connected to a conduit II for cold water and a conduit I2 for hot water via check valves I3 and I4, respectively, and a mixer I5 with control means I6 is connected to said conduits. The outlet of the mixer is connected via a shut-off cock I7 to a water container I8 located in the upper portion of the apparatus. The water container has an outlet conduit I9 connected to a bottom thereof, to which a conduit 20 is connected, said conduit being connected via a shut-off valve 2I to a nipple 22 available at the outside of the housing I0. An overflow 23 for the water container is connected to the conduit I9 which opens via a shut-off valve 24 in a drainage system 25.

Upstream of the valve 2I a conduit 26 having a shut-off valve 27 is connected to the conduit 20, and said conduit 26 opens in the water container at a location above the uppermost water level in the water container defined by the overflow 23. Downstream of the valve 2I a conduit 28 having a shut-off valve 29 is connected to the conduit 20, and said conduit 29 extends from the cold water conduit 3I which is also connected by a conduit 30 having an adjustable choke member 3I the control means of which is indicated at 32, and a shut-off valve 33, to an injector (water type suction device) 34 having an outlet to the drainage system 25. The suction conduit 35 of the injector is connected via a shut-off valve 36 and a check valve 37 to a nipple 38 which is available at the outside of the housing I0 and is located adjacent the nipple 22.

Transparent hoses 39 and 40 are detachably connected to the nipples 22 and 38, respectively, said hoses extending to a rectal nozzle 4I.

The rectal nozzle 4I is circular in the cross sections thereof and is anatomically shaped in the longitudinal direction in order that the insertion into the rectum will be facilitated and optimum sealing against the intestinal wall and the rectal muscle and - when patients having undergone stomi-operation are treated - against the skin and the intestinal wall at the intestinal outlet will be obtained.

The nozzle has a through internal passage 42 which in the forward portion 43 opens into a centrally positioned supply opening 44 and in the sides of said portion opens into two (or more) discharge openings 45. In the rear portion 46 two connection tubes 47 and 48 are provided communicating with the passage 42, and the two hoses 39 and 40 are connected each to one of these connection tubes.

The forward portion of the rectal nozzle is shaped so as to simplify and facilitate the application of the nozzle at and during the treatment. The forward, outside portion of the nozzle is smoothly curved so as to prevent damage of the intestine and the rectal muscle and to lower the level of pain when the nozzle is being inserted into the rectum and prevent reflectory contraction of the rectal muscle. The forward portion 43 of the nozzle has a larger diameter than the central portion 49 and, therefore, the openings 45 can be made relatively large so as to pass larger fractions of the intestinal content without the risk of stoppage. When the forward, larger portion has passed the rectal muscle which then encircles the following narrower portion 49, this muscle will be less strained but prevents simultaneously the nozzle from spontaneously slipping out of the intestine. If the nozzle is used for intestinal irrigation of stomi-operated patients the length of the thicker portion 43 provides an indication of how far the nozzle should be inserted into the intestinal opening. The rear portion 46 of the nozzle has a substantially larger diameter than the central portion 49 and the forward portion 43. From the central portion the diameter is increased by a smooth transition 50 to the largest diameter which approaches the double diameter of the central portion. The function of this enlargement is that the portion at the smooth transition from a small to a large diameter when said enlarged portion is pressed against the rectal opening will form a tight connection at said opening. This is of particular importance in treating elderly patients whose rectal functions often are weakened.

Preferably, the rectal nozzle is made of a transparent plastics material and suitably is a one-way product. When the nozzle is produced the two fixedly attached connection tubes 47 and 48 are formed as nipples for hose connection.

Necessary control means for the cock and the shut-off valves as well as necessary check means are provided on the outside surface of the housing 10. Thus, there are provided an operating handle 127 for the air valve 27, an operating handle 133 for the valve 33 used for applying a negative pressure in the conduit 35, a knob 132 connected to the adjustment means 32 for adjusting the negative pressure in the conduit 35, an operating handle 121 for the valve 21 which can be marked "To patient", an operating handle 136 for the valve 36, which can be marked "From patient", an operating handle 117 for the water cock 17, a knob 116 for adjusting the control means 16 for the mixer for setting a suitable temperature of the water supplied from the mixer, an operating handle 124 for the valve 24 for emptying the water container, and an operating handle 129 for the valve 29. Additionally, there is provided on the apparatus a level meter 51 for indicating the water level in the water container and a digital thermometer 52 for indicating the temperature of the water in the water container.

When enema is performed by using the apparatus described the procedure will be as follows:

Starting from a condition wherein the water cock 17 and all valves are closed the negative pressure to be maintained in the conduit 35 initially is adjusted. The hose 40 (detached from the nozzle) is immersed into a bucket with water valves 33 and 36 then being opened. By means of the injector 34 a negative pressure is developed in the hose 40 a water column being sucked up into said hose. The water column can be seen through the transparent hose, and at the knob 132 the water flow through the injector is adjusted such that the desired negative pressure will be obtained; the distance from the water column to the nipple 38 can be used as a measure of the negative pressure. For example it may be prescribed for the use of the apparatus that this distance in the normal case should be about 10 cm.

The water container 18 is filled with body temperated water by opening the cock 17 with the knob 116 adjusted to the suitable temperature which is read on the thermometer 52 the water container initially being flushed by the valve 24 being maintained in the open position. When the rectal nozzle 41 has been inserted into the rectum of the patient with the two hoses 39 and 40 connected to the nozzle and the apparatus, the valve 27 in the air conduit 26 is opened and then the valve 21 is opened, water flowing by gravity from the water container through the conduits 19 and 20, the hose 39 and the nozzle 41 into the intestine of the patient. At the same time the water is airated via the conduit 26 the air being preheated by being taken from the water container 18 above the surface of the body hot water contained therein. The air contributes to an expansion of the intestine so that walls and intestinal cavities will be available for the liquid, which means that the cleansing effect will be optimated and that the treatment time as a consequence thereof can be reduced in relation to that required at conventional enema. When 1/2 - 1 l water has been supplied the valve 21 is closed and the valve 36 in the conduit 35 is opened, water and intestinal contents being evacuated through the nozzle 41, the hose 40 and the conduit 35 to the drainage system 25 via the injector 34 under the influence of the negative pressure generated in said injector. This procedure is repeated until the desired degree of cleansing has been obtained, which can easily be checked by observing the colour of the material which is evacuated through the transparent hose 40. Then, the valve 36 is closed and the nozzle 41 is removed from the patient. The water container 18 is emptied by opening the valve 24. The valves 27 and 33 are closed.

In order to clean the apparatus the nipples 22 and 38 are short circuited by connecting a short hose connection indicated by dot-and-dash lines at 53 between the nipples. The valve 36 is opened as is the valve 29 cold water flushing through the conduits 20 and 35 via the hose 53 to the drainage system 25. When the valve 29 has been closed the valves 2l and 33 are open for evacuating the conduits and the hose. Then, all valves are closed, and the apparatus is ready to be used again.

Regular disinfection of the apparatus should be effected, and this is done in the following manner.

With the hose 53 connected to the nipples 22 and 38 and with the valve 2l open the water container l8 is filled with water by opening the cock l7 and admixing a disinfection agent into the water in the container. When the mixture has been left for an hour or so the valves 24 and 36 are opened, the conduits l9, 20 and 35 being flushed with disinfection liquid. The procedure is repeated some times with water only from the water container l8 the cleaning program described above then being run through in order to prepare the apparatus for enema.

The check valve 37 guarantees that cold water at water tap pressure cannot be supplied to the patient via the rectal nozzle 4l if the injector 34 should fail or stoppage should arise in the drainage system 25. In order that cold water at water tap pressure also cannot be supplied to the patient by opening the valve 29 when the nozzle 4l is connected to the patient, the operating handle l29 of the valve 29 should be located so that it is separated from other control handles regularly used in treating a patient by means of the apparatus, as is also disclosed in FIG 2, and the access thereof should be more difficult than in case of other operating handles and, preferably, the handle should be marked with a particular colour, for example painted in red colour.

As will be seen the apparatus of the invention allows enema in a closed system in a simple and effective manner the apparatus being operated by simple manipulation, and that the apparatus in a simple manner can be adjusted for use and can be cleaned and disinfected in order to satisfy strict hygienic requirements at the level maintained nowadays in the medical care. The cleansing procedure can be effected more rapidly and with a better result than when conventional enema is used. Summarizing, there are thus obtained by the invention practical, hygienical, time related and economic advantages, the situation of the patient at the same time being less degrading as compared with the conventional enema method.

**Claims**

1. Apparatus for gastro-intestinal irrigation and cleansing of humans and animals comprising a water container (18) having cold and hot water connections (11, 12, 15) for the supply of temperated water to the container, and a nozzle (41) which can be connected by hoses (39, 40) over valve controlled conduits (20, 35) to the water container for supply of water to the nozzle from the water container by gravity and to a drain (25) for discharging flowing material from the nozzle to the drain, the cold water connection (11) being connected via a shut-off valve (29) to the conduit (20) from the water container (18) to the nozzle (41) and via a shut-off valve (33) to the drain (25), the nozzle hoses being transparent and detachably connected to the valve controlled conduits (20, 35) from the water container and to the drain, respectively, whereby in a conduit (30) connecting the cold water connection (11) to the drain (25) there is provided separate adjustable choke means (31), **characterized** in that downstream of the choke mans (31), there is provided an injector (34) having a suction inlet thereof connected to the conduit (35) from the nozzle (41) to the drain (25) for connection of the nozzle to the drain via said injector so as to create a suction in the conduit (35) from the nozzle to the drain (25), a check valve (37) being provided in th drain conduit (35) of the nozzle to prevent flow to the nozzle (41) through said conduit, the nozzle hoses being connected to the valve controlled conduits (20, 35) by means of hose couplings (22, 38) available externally on a housing (10) enclosing the rest of the apparatus.

2. Apparatus as in claim 1 wherein operating means (129) for the shut-off valve in the cold water connection (11) to the supply conduit (20) of the nozzle (41) is separated from operating and control means for other functional elements of the apparatus, and preferably the access thereof is more difficult than that of said other elements.

3. Apparatus as in claim 1 or 2 wherein the cold water connection (11) to the supply conduit (20) of the nozzle (41) is located downstream of the shut-off valve (21) for the connection between the water container (18) and the nozzle.

4. Apparatus in any of claims 1 to 3 wherein a valve controlled conduit (26) for supply of gas, preferably air, to the water flow through the connection between the water container (18) and the nozzle (41) is connected with said connection upstream of the shut-off valve (21) therein.

5. Apparatus as in claim 4 wherein said conduit (26) opens into the water container (18) above a maximum water level therein defined by an overflow (23).

**Patentansprüche**

1. Verfahren zum Spülen und Reinigen des Magen-Darm-Traktes von Menschen und Tieren, die einen Wasserbehälter (18) mit Kalt- und Warmwasserverbindungen (11, 12, 15) zur zufuhr von temperiertem Wasser zum Behälter, und eine Düse (41) umfaßt, die durch Schläuche (39, 40) über ventilgesteuerte Leitungen (20, 35) mit dem Wasserbehälter verbunden werden kann, um Wasser durch die Schwerkraft vom Wasserbehälter zur Düse und zum Ablauf (25) zu leiten, um das aus der Düse strömende Material zum Ablauf abzugeben, wobei die Kaltwasserverbindung (11) durch ein Absperrventil (29) mit der Leitung (20) vom Wasserbehälter (18) zur Düse (41) und durch ein Absperrventil (33) mit dem Ablauf (25) verbunden ist, wobei die Schläuche der Düse transparent und lösbar mit den ventilgesteuerten Leitungen (20, 35) vom Wasserbehälter bzw. zum Ablauf verbunden sind, wodurch in der Leitung (30), die die Kaltwasserverbindung (11) mit dem Ablauf (25) verbindet, eine separat regelbare Drosseleinrichtung (31) geschaffen wird, dadurch **gekennzeichnet**, daß stromabwarts der Drosseleinrichtung (31) ein Injektor (34) vorgesehen ist, dessen Ansaugeinlaß mit der Leitung (35) von der Düse (41) zum Ablauf (25) verbunden ist, um die Düse durch den Injektor mit dem Ablauf zu verbinden, so daß in der Leitung (35) von der Düse zum Ablauf (25) eine Saugwirkung erzeugt wird, wobei das Regelventil (37) in der Ablaufleitung (35) der Düse vorgesehen ist, um die Strömung durch die Leitung zur Düse (41) zu verhindern, wobei die Schläuche der Düse durch Schlauchverbindungen (22, 38), die außerhalb auf dem Gehäuse (10) zugänglich sind, das den Rest der Vorrichtung umgibt, mit den ventilgesteuerten Leitungen (20, 35) verbunden sind.

2. Vorrichtung nach Anspruch 1, worin die Bedienungseinrichtung (129) für das Absperrventil in der Kaltwasserverbindung (11) zur zufuhrleitung (20) der Düse (41) von der Bedienungs- und Regeleinrichtung für die anderen funktionellen Elemente der Vorrichtung getrennt ist, und der zutritt zu dieser vorzugsweise schwieriger als der zu den anderen Elementen ist.

3. Vorrichtung nach Anspruch 1 oder 2, worin die Kaltwasserverbindung (11) zur zufuhrleitung (20) der Düse (41) stromabwärts des Absperrventils (21) für die Verbindung zwischen dem Wasserbehälter (18) und der Düse angeordnet ist.

4. Vorrichtung nach einem der Ansprüche 1 bis 3, worin die ventilgesteuerte Leitung (26) zur zufuhr von Gas, vorzugsweise Luft, zum Wasserstrom durch die Verbindung zwischen dem Wasserbehälter (18) und der Düse (41) mit der Verbindung stromaufwärts des Absperrventils (21) verbunden ist.

5. Vorrichtung nach Anspruch 4, worin die Leitung (26) in den Wasserbehälter (18) oberhalb des maximalen Wasserniveaus in diesem Behälter mündet, das durch den Überlauf (23) definiert wird.

**Revendications**

1. - Appareil servant à effectuer une irrigation et un lavage gastro-intestinaux de patients et d'animaux, comprenant un réservoir d'eau (18) présentant des liaisons d'eau froide et chaude (11, 12, 15) pour l'amenée d'eau à température modérée au réservoir, et une buse (41) qui peut être reliée, par des tuyaux (39, 40) donnant sur des conduits commandés par soupape (20, 35), au réservoir d'eau, pour l'amenée d'eau à la buse, depuis le réservoir d'eau, sous l'effet de la gravité, et à un drain (25) pour décharger la matière s'écoulant depuis la buse, vers le drain, la liaison d'eau froide (11) étant reliée, via une vanne d'arrêt (29), au conduit (20) allant du réservoir d'eau (18) à la buse (41) et, via une vanne d'arrêt (33), au drain (25), les tuyaux de buse étant transparents et reliés de manière démontable aux conduits commandés par soupape (20, 35), respectivement depuis le réservoir d'eau et le drain, de manière que, dans un conduit (30) reliant la liaison d'eau froide (11) au drain (25), il soit prévu un moyen d'étranglement réglable (31) séparé, caractérisé en ce que, en aval du moyen d'étranglement (31), il est prévu un injecteur (34) dont l'entrée d'aspiration est reliée au conduit (35) allant de la buse (41) au drain (25), pour assurer la liaison de la buse au drain via ledit injecteur, de manière à créer une aspiration dans le conduit (35) allant de la buse au drain (25), un clapet anti-retour (37) étant monté dans le conduit de drain (35) de la buse, pour empêcher un écoulement vers la buse (41) à travers ledit conduit, les tuyaux de buse étant reliés aux conduits commandés par soupape (20, 35), au moyen de raccords de tuyau (22, 38) accessibles à l'extérieur et montés sur un boîtier (10) enfermant le restant de l'appareil.

2. - Appareil selon la revendication 1, dans lequel un moyen d'actionnement (129) pour la vanne d'arrêt située dans la liaison d'eau froide (11), vers le conduit d'amenée (20) de la buse (41), est séparé du moyen d'actionnement et de commande pour d'autres moyens fonctionnels de l'appareil, son accès étant de préférence plus difficile que celui desdits autres éléments.

3. - Appareil selon la revendication 1 ou 2, dans lequel la liaison d'eau froide (11) vers le conduit d'amenée (20) de la buse (41) est située en aval de la vanne d'arrêt (21), pour assurer la liaison entre le réservoir d'eau (18) et la buse.

4. - Appareil selon l'une quelconque des revendications 1 à 3, dans lequel un conduit commandé par soupape (26) pour l'amenée d'un gaz, de préférence de l'air, à l'écoulement d'eau passant dans la liaison entre le réservoir d'eau (18) et la buse (41), est relié à ladite liaison en amont de la vanne d'arrêt (21), située dans cette dernière.

5. - Appareil selon la revendication 4, dans lequel ledit conduit (26) débouche dans le réservoir d'eau (18), au-dessus d'un niveau d'eau maximal défini dans ce dernier, par un conduit de trop-plein (23).

FIG. 1

**FIG. 2**

FIG. 3

FIG. 4

FIG. 5

FIG. 6